# EUROPEAN PATENT SPECIFICATION

(11) **EP 3 593 765 B1**
(45) Date of publication and mention of the grant of the patent: **04.05.2022**
(21) Application number: 19185766.3
(22) Date of filing: 11.07.2019
(51) Int. Cl.: A61F 2/80

(54) **PROSTHETIC SOCKET FOR A CONNECTION TO A LOWER LIMB PROSTHESIS**
PROTHESENSCHAFT FÜR EINE VERBINDUNG MIT EINER UNTERSCHENKELPROTHESE
EMBOÎTURE DE PROTHÈSE POUR UNE CONNEXION À UNE PROTHÈSE DE MEMBRE INFÉRIEUR

(30) Priority: 13.07.2018 NL 2021306
(43) Date of publication of application: 15.01.2020
(73) Proprietor: Livit Orthopedie B.V., 1014 BB Amsterdam (NL)
(72) Inventor: De Kiefte, Bertus Evert, 3315 WD DORDRECHT (NL); Ravestein, Maurits Willem, 2651 SZ BERKEL EN RODENRIJS (NL)
(74) Representative: Luten, Martin Haaije

(56) References cited:
- WO-A1-2013/071308
- AU-B2- 686 514
- US-A1- 2010 036 505

## Description

The present invention relates to a prosthetic arranged for connection to a lower limb prosthesis, wherein the prosthetic socket comprises a receptacle for receiving a stump of a patient, wherein the receptacle comprises a bottom for receiving a distal end of the stump and an inner side wall for at least partially enclosing said stump. Described is further a method for manufacturing such a prosthetic socket.

For connecting a lower limb prosthesis to a patient, a prosthetic socket is typically used. The socket is arranged on the stump and connects to the actual prosthesis, for instance an artificial lower leg or lower arm. Such a socket typically has receptacle, for instance having a cuplike shape, wherein the bottom of the cup is arranged for receiving the stump of the patient. This limits relative movement in the distal direction. The inner side wall of the cup extending from the bottom is designed to prevent any lateral movement of the stump. The shape of the cup or receptacle is designed to closely match the anatomy of the stump of the patient to prevent or at least reduce any relative movement between the stump and the socket.

It is known fabricate a socket by making a cast of the amputee's residual limb. The socket, specifically the receptacle thereof, can then be shaped to closely fit the shape of the cast. As an alternative, the socket can be designed using three dimensional imaging techniques. The socket, in particular the receptacle thereof, can for instance be designed by computer aided design based on three dimensional image data obtained by medical imaging. To fabricate the socket based on the design, a three dimensional printing technique may be used.

It is an goal, amongst other goals, to provide a further improved prosthetic socket. This goal, amongst other goals, is met by a prosthetic socket according to claim 1. Specifically, to meet this goal, the above mentioned prosthetic socket is characterized in that the inner side wall is defined by a plurality of wall portions and wherein the prosthetic socket comprises an adjustment mechanism for adjusting the relative position of at least two of the wall portions. This allows adjusting the shape of the inner side wall. It has been found that the size of stump, in particular in a transverse plane, i.e. a plane perpendicular the longitudinal axis of the stump, varies over time. By providing an adjustment mechanism, the positions of at least two wall portions of the inner side wall can be set in accordance with the shape and/or size of the stump. It is then no longer necessary to design and manufacture a new socket to adapt to varying sizes of the stump.

The invention is specifically advantageous for use in a lower limb prosthesis, although the invention is not limited to this. A socket for a lower limb needs to be applied to a stump having relatively hard tissue compared to for instance upper limbs. These limbs tend to have more fat or muscle tissue onto which a socket can be clamped. Fitting a socket to relative stiff tissue requires a tighter fit of the socket. WO2013071308 describes modular prosthetic sockets.

The wall portions are preferably movable in a direction having a component in the radial direction, seen with respect to the longitudinal axis of the stump. By moving at least one of the wall portions in the transverse plane, changes in shape and/or size of the stump in this plane can be compensated for.

In order to compensate for changes in diameter or perimeter of the stump, which are most critical, the adjustment mechanism is arranged for adjusting a diameter or perimeter of the inner side wall of the receptacle according to a preferred embodiment. Preferably, the two wall portions, the relative position of which is adjustable, extend at different positions along the diameter of the receptacle. This allows an efficient adaptation.

The size of the stump may change locally. In order to compensate for local variations in size, it is preferred if the adjust mechanism is arranged to adjust the position of a plurality of wall portions. The respective wall portions, arranged to engage respective locations of the stump, can then be adjusted accordingly.

The inner side wall is typically designed to have a good fit to provide a stable connection for the receptacle. The inner side wall may thereto extend along the complete perimeter of the stump. This is however not necessary. Preferably, the inner side wall is formed from a plurality of separate wall portions which together enclose the stump in the transverse plane for a stable fit. This improves the comfort for the wearer of the socket. Seen along the perimeter, spaces may extend between the wall portions. An efficient and comfortable socket is obtained when the two wall portions extend at approximately diametric opposite locations of the inner side wall.

It was found that the size of the stump not only changes gradually over time, but also within day or even within a day. To be able to adjust for these changes, it is preferred if the adjustment mechanism is manually operable, for instance using a knob. The user can thus adjust the position of the wall portions, for instance adjust the diameter of the inner side wall of the receptacle.

It is preferred if the adjustment mechanism defines a maximum diameter or perimeter of the inner side wall. The adjustment mechanisms may for instance limit the movement of the wall portions in a direction radially outward. Movement inwardly is then limited by the stump. The wall portions, preferably extending along the perimeter of the stump, may for instance be connected using a connecting member. By adjusting the length of the connecting member, the relative position of the wall portions can be adjusted. The connecting member may for instance comprise a tensioning member such as a cable or strip. The connecting member preferably extends around the perimeter of the inner side wall or the receptacle, thereby defining the maximum diameter. Preferably, the connecting member extends around or along the wall portions. The diameter is then determined at the locations of the wall portions, i.e. the parts for stably contacting the stump. An easy to use system is obtained if the adjustment mechanism further comprises a turning knob for adjusting the length of the connecting member. It is preferred if the actuating device, such as the knob, extends between the wall portions.

In order to efficiently receive the connecting member, it is preferred if the receptacle comprises at least one channel for receiving the connecting member. Preferably, the parts comprising the respective wall portions may be provided with a channel for receiving the connecting member, such that the connecting member interconnects the respective parts having the wall portions. By adjusting the length of the connecting member, the respective positions of the wall portions may be accordingly adjusted.

In order to allow an efficient movement of the wall portions for adjusting to a varying size of the stump, it is preferred if the socket comprises at least one flexible section interconnecting a wall portion to the bottom. The wall portion, or any part comprising the wall portion, is then flexibly attached to the bottom, thereby allowing a relative movement of the wall portion. The wall portion may be movable with respect to the bottom and any wall portion fixedly connected to said bottom. Preferably, each of the wall portions is movable as mentioned above, wherein each of the wall portions, or the parts comprising said wall portions, is flexibly attached to the bottom.

Preferably, the flexible section is arranged to allow movement of the wall portions in a radial direction, seen with respect to the longitudinal axis of the stump, which is substantially perpendicular to the surface of the bottom. This allows adjustment of the wall portion to varying size in respect of diameter of the stump. The flexible section may comprise a hinge or other type of joint. It is however preferred if the flexible section is manufactured from the same material as the socket. The flexible section may for instance be formed thinner or otherwise shaped to introduce flexibility. It is preferred that the flexible section is more flexible or has a smaller young's modulus than the wall portions and/or the bottom. The bottom and the wall portions are then arranged to receive and engage the stump, whereas the flexible section allows relative movement to ensure the close fit.

According to a further preferred embodiment, the socket comprises a plurality of fin shaped members connected to the bottom, wherein the inner surfaces of the fin shaped members comprise the wall portions. The fin or wing shaped members extend under an angle with respect to the bottom, thereby forming a cup shaped receptacle for the stump. Preferably, the fin shaped members are connected to the bottom using a flexible section as mentioned above. This allows efficient movement of the fin shaped members.

An efficient configuration is obtained if fin shaped members extend radially from the bottom. The socket is hereby star shaped, with the bottom in the center and the fin shaped members extending radially therefrom. The proximal parts of the fin shaped member are bent upwardly, forming the cup as mentioned above. It is preferred if the socket comprises at least three fin shaped members, preferably exactly three. This provides an stable fit between the socket and the stump.

It is preferred if the fin shaped members are interconnected using the connecting member as mentioned above. According to a preferred embodiment, the connecting member extends between the fin shaped members at the location of the wall portions. The connecting member, for instance a cable, cord or strip, then limits any movement of the wall portions of the fin shaped members in a direction radially outwardly. It is then again preferred that the channel in a fin shaped member extends at the location of the wall portion.

The present invention is further illustrated by the following Figures, which show a preferred embodiment of the device according to the invention, and are not intended to limit the scope of the invention in any way, further a method is shown, wherein:
- Figure 1 show the socket in a front view;
- Figure 2 shows the socket with schematically indicated limb in back view;
- Figure 3 shows a top view of the socket;
- Figure 4 shows a cross-sectional view of the socket including limb; and
- Figures 5a-c schematically show the different steps of adjusting the socket to a limb.

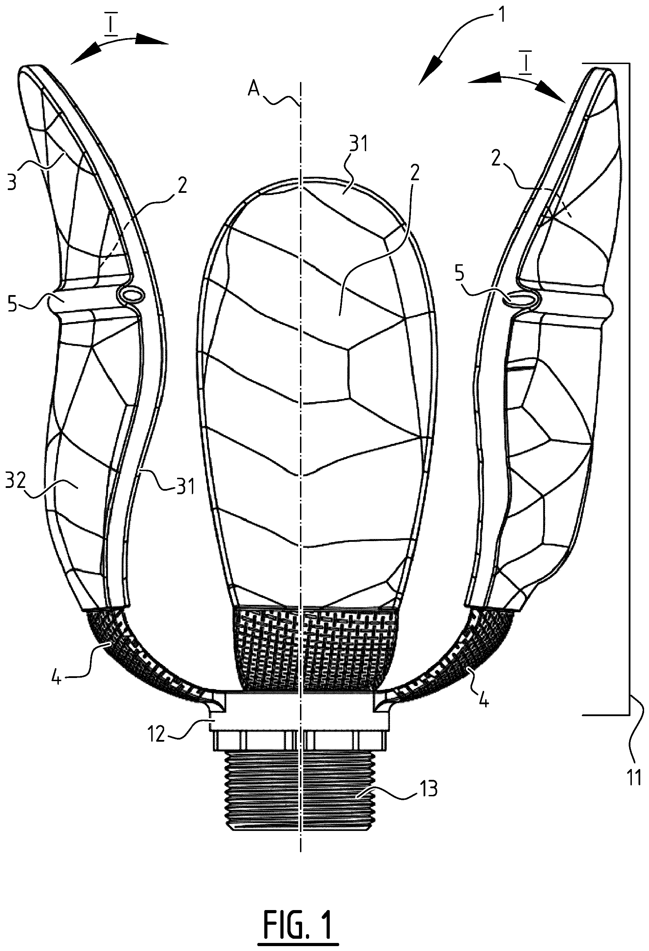

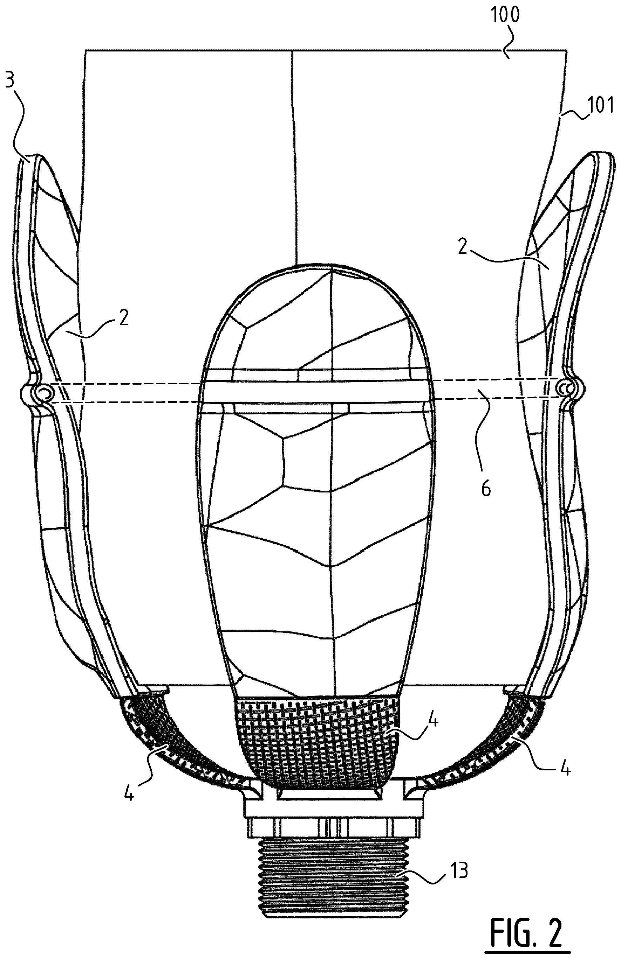

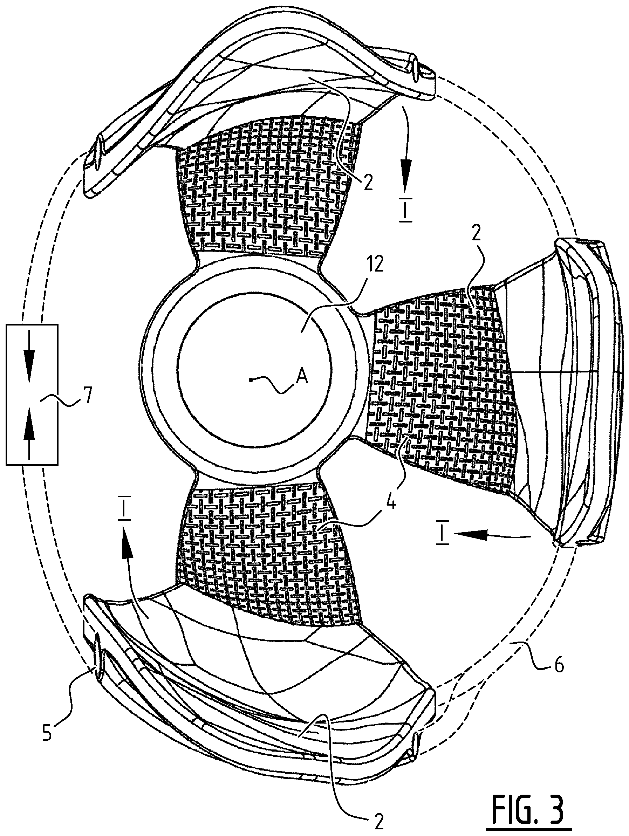

In figures 1 - 4 a prosthetic socket 1 for connection to a lower limb prosthesis is shown. The socket comprises a cup shaped receptacle 11, defined by a bottom 12 (see figure 3 for a top view) and side walls defined by the inner surfaces 31 of fin shaped members 3. The receptacle is arranged for receiving the stump 100 (see for instance figure 2), whereas on the lower side of the bottom 12 a prosthesis connector 13 is provided, in this example a bushing provided with threading. A prosthetic lower leg can for instance be screwed on the connector 13. For the prosthesis to function correctly and in a comfortable manner, it is important that the receptacle 11 closely fits to the stump 100 with al little as play as possible.

The inner surfaces 31 of the fin shaped members 3 define wall portions 2 which are designed to mate with the perimeter 101 of the stump 100. The inner surfaces 31 and specifically the wall portions 2 arranged to contact the stump 100, may be formed patient specifically, for instance based on a three dimensional model of the patient obtained by a three-dimensional imaging technique such as MRI. The wall portions 2 may for instance be shaped to closely follow the outer contour 101 of the stump 100.

As best seen in the top view of figure 3, the three fin shaped members 3 extend radially from the bottom A, seen with respect to the longitudinal axis A (indicated in figures 1 and 3. The three fin shaped members 3, in particular the wall portions 2 on the inner surfaces thereof, thereby form a receptacle which is arranged to receive the stump 100 of a patient in a stable manner. From the figures, it is clear that although the wall portions 2 extend along various parts of the perimeter 101 of the stump 100, the stump 100 is not contacted along the complete perimeter 101 due to the space between the fin shaped members 3. This improves the comfort for the patient.

The fins 3 are connected to the bottom 12 via flexible sections 4. The socket 1 is manufactured from a relatively rigid plastic, while the flexible sections 4 are provided cuts (best seen in figure 3) to introduce flexibility to the flexible sections 4. Due to these sections 4, the fins 3 are allowed to move radially (indicated with the arrows I in figure 1) with respect to the longitudinal axis A.

Provided in the fins 3, in this example at the locations of the wall portions 2 specifically mating with the perimeter of the stump 100, are channels 5. Channels 5 define a passage for a cord 6 (see figures 2 and 3) interconnecting the fins 3 preferably at a proximal location with respect to the bottom 12. The end of the cord 6 are interconnected in a turning knob 7, which upon rotation of the knob 7 can change the length of the cord 6 forming the loop, or more generally interconnects the fins 3.

The knob 7 and cord 6 are thus arranged to adjust the relative positions of the wall portions 2 of the fins 3. Shortening the cord 6 will result in a movement of the fins towards the axis A, while increasing the length allows a movement away from the axis A. The diameter of the cup shaped receptacle 11 can thus be adjusted. More specifically, the receptacle 11 can be adjusted such that a stump 100 with varying perimeter 101 can be closely received with minimal or no play.

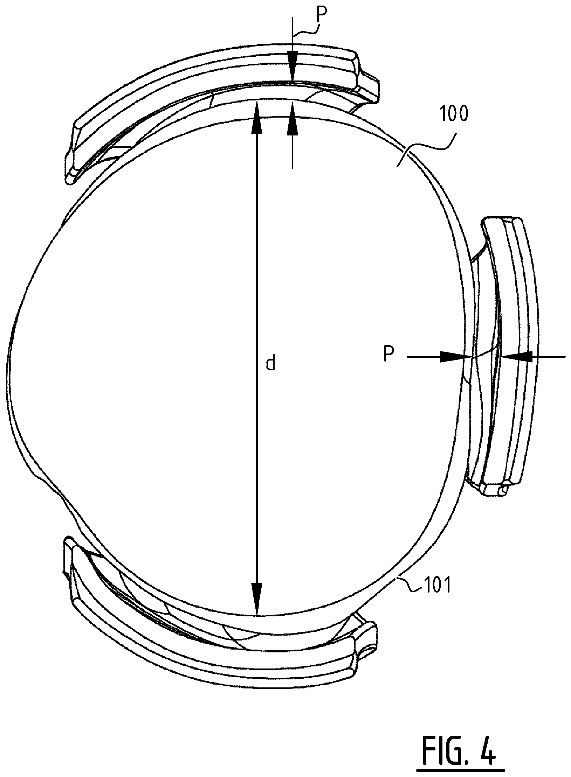

It was found, that the diameter d (see figure 4) and the perimeter 101 of a stump varies. Even within a day or a couple of days, this variation is substantial. This variation may lead to play P (indicated in figure 4) between the fins 3. As mentioned above, play between the stump 100 and the socket 1, in particular the wall portions 2 of the fins 3 thereof, is detrimental to the usability and comfort for the patient.

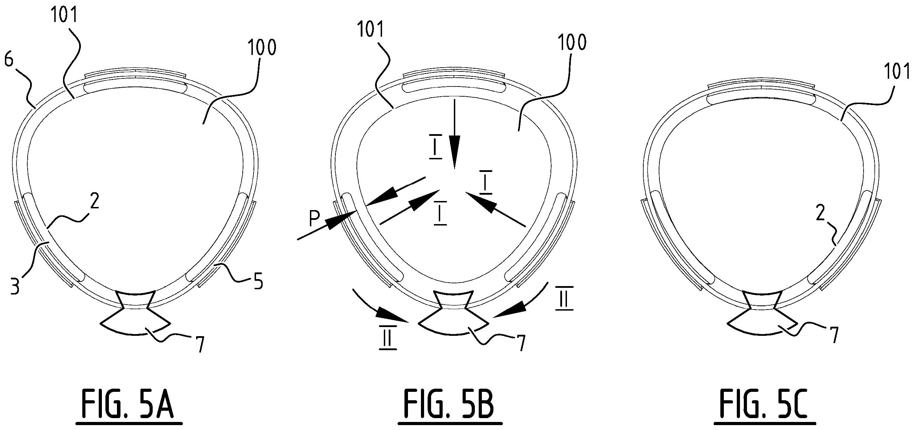

As an example and with reference to figure 5a, although initially the fit between the wall portions 2 of the fins 3 and the perimeter 101 of the stump 100 may be good, the size (in particular the perimeter) of the stump 100 may decrease over time. This is indicated in figure 5B, again indicating the play P between the perimeter 101 of the stump 100 and the wall portions 2 of the fins 3. However, by actuating the mechanism 7, in this example a turning knob, the cable 6 is tensioned (indicated with arrows II), thereby shortening the loop and thus moving the wall portions 2 relative to each other. The movement of the wall portions 2 radially inwardly towards the stump 100 is indicated with arrows I.

The user can manually operate the knob 7 up to a situation wherein he/she feels that the wall portions 2 exert a sufficient clamping action on the stump 100, while not causing discomfort. It will be appreciated that by turning the knob 7 the other way, the size of the receptacle may be increased if needed.

The present invention is not limited to the embodiment shown, but extends also to other embodiments falling within the scope of the appended claims.

## Claims

1. Prosthetic socket (1) arranged for connection to a lower limb prosthesis, wherein the prosthetic socket (1) comprises a receptacle (11) for receiving a stump (100) of a patient, wherein the receptacle (11) comprises a bottom (12) for receiving a distal end of the stump and an inner side wall (31) for at least partially enclosing said stump (100), wherein the inner side wall (31) is defined by a plurality of wall portions (2) and wherein the prosthetic socket (1) comprises an adjustment mechanism (6,7) for adjusting the relative position of at least two of the wall portions (2), **characterized in that** the prosthetic socket (1) further comprises at least one flexible section (4) interconnecting a wall portion (2) to the bottom (12), wherein the flexible section (4) is more flexible than the bottom (12) and/or the wall portion (2).

2. Prosthetic socket (1) according to claim 1, wherein the flexible section (4) is manufactured from the same material as the socket (1).

3. Prosthetic socket (1) according to claim 2, wherein the flexible section (4) is formed thinner or shaped otherwise to introduce the flexibility.

4. Prosthetic socket (1) according to claim 1, 2 or 3, wherein the adjustment mechanism (6,7) is arranged for adjusting a diameter of the inner side wall (31) of the receptacle (11).

5. Prosthetic socket (1) according to any of the preceding claims, wherein the adjust mechanism (6,7) is arranged to adjust the position of a plurality of wall portions (2).

6. Prosthetic socket (1) according to any of the preceding claims, wherein the at least two wall portions (2) extend at approximately diametric opposite locations of the inner side wall (31).

7. Prosthetic socket (1) according to any of the preceding claims, wherein the adjustment mechanism (6,7) is manually operable, preferably using a knob (7).

8. Prosthetic socket (1) according to any of the preceding claims, wherein the adjustment mechanism (6,7) comprises a connecting member (6) extending between at least two wall portions (2), wherein the adjustment mechanism (6,7) is arranged to adjust the length of said connecting member (6).

9. Prosthetic socket (1) according to claim 7, wherein said tensioning member (6) extends around the perimeter of the receptacle (11) and/or wherein the adjustment mechanism (6,7) further comprises a turning knob (7) for adjusting the length of the connecting member (6).

10. Prosthetic socket (1) according to claim 8 or 9, wherein the receptacle (11) comprises at least one channel (5) for receiving the connecting member (6).

11. Prosthetic socket (1) according to any of the preceding claims, wherein the socket (11) comprises a plurality of fin shaped members (3) connected to the bottom (12) using the at least one flexible section (4), wherein the inner surfaces (31) of the fin shaped members (3) comprise the wall portions (2).

12. Prosthetic socket (1) according to claim 11, wherein the fin shaped members (3) extend radially from the bottom (12).

13. Prosthetic socket (1) according to claim 11 or 12, comprising at least three fin shaped members (3).

14. Prosthetic socket (1) according to claim 8 and any of the claims 11, 12 or 13, wherein the connecting member (6) extends between the fin shaped members (3) at the location of the wall portions (2).

15. Prosthetic socket (1) according to claim 10 and 14, wherein the channel (5) in a fin shaped member (3) extends at the location of the wall portion (2).

## Patentansprüche

1. Prothesenschaft (1), der zur Verbindung mit einer Prothese der unteren Gliedmaßen ausgebildet ist, wobei der Prothesenschaft (1) eine Aufnahme (11) zur Aufnahme eines Stumpfes (100) eines Patienten umfasst, wobei die Aufnahme (11) einen Boden (12) zur Aufnahme eines distalen Endes des Stumpfes und eine innere Seitenwand (31) zum zumindest teilweisen Umschließen des Stumpfes (100) aufweist, wobei die innere Seitenwand (31) durch eine Mehrzahl an Wandabschnitten (2) definiert ist und wobei der Prothesenschaft (1) einen Einstellmechanismus (6, 7) zum Einstellen der relativen Position von mindestens zwei der Wandabschnitte (2) aufweist, **dadurch gekennzeichnet, dass** der Prothesenschaft (1) ferner zumindest einen flexiblen Abschnitt (4) aufweist, der einen Wandabschnitt (2) mit dem Boden (12) verbindet, wobei der flexible Abschnitt (4) flexibler ist als der Boden (12) und/oder der Wandabschnitt (2).

2. Prothesenschaft (1) nach Anspruch 1, wobei der flexible Abschnitt (4) aus dem gleichen Material gefertigt ist wie der Schaft (1).

3. Prothesenschaft (1) nach Anspruch 2, wobei der flexible Abschnitt (4), um die Flexibilität herzustellen, dünner ausgebildet oder anderweitig geformt ist.

4. Prothesenschaft (1) nach Anspruch 1, 2 oder 3, wobei der Einstellmechanismus (6, 7) zum Einstellen eines Durchmessers der inneren Seitenwand (31) der Aufnahme (11) angeordnet ist.

5. Prothesenschaft (1) nach einem der vorherigen Ansprüche, wobei der Einstellmechanismus (6, 7) so angeordnet ist, dass er die Position einer Mehrzahl an Wandabschnitten (2) einstellt.

6. Prothesenschaft (1) nach einem der vorherigen Ansprüche, wobei sich die zumindest zwei Wandabschnitte (2) an etwa diametral gegenüberliegenden Stellen der inneren Seitenwand (31) erstrecken.

7. Prothesenschaft (1) nach einem der vorherigen Ansprüche, wobei der Einstellmechanismus (6, 7) manuell zu betätigen ist, vorzugsweise mittels eines Knopfes (7).

8. Prothesenschaft (1) nach einem der vorherigen Ansprüche, wobei der Einstellmechanismus (6, 7) ein Spannelement (6) aufweist, das sich zwischen zumindest zwei Wandabschnitten (2) erstreckt, wobei der Einstellmechanismus (6, 7) angeordnet ist, um die Länge des Spannelementes (6) einzustellen.

9. Prothesenschaft (1) nach Anspruch 7, wobei sich das Spannelement (6) um den Umfang der Aufnahme (11) erstreckt und/oder wobei der Einstellmechanismus (6, 7) zum Einstellen der Länge des Spannelementes (6) ferner einen Drehknopf (7) aufweist.

10. Prothesenschaft (1) nach Anspruch 8 oder 9, wobei die Aufnahme (11) mindestens einen Kanal (5) zur Aufnahme des Spannelementes (6) aufweist.

11. Prothesenschaft (1) nach einem der vorherigen Ansprüche, wobei der Schaft (1) eine Mehrzahl an flossenförmigen Elementen (3) aufweist, die unter Verwendung des zumindest einen flexiblen Abschnittes (4) mit dem Boden (12) verbunden sind, wobei die Innenflächen (31) der flossenförmigen Elemente (3) die Wandabschnitte (2) umfassen.

12. Prothesenschaft (1) nach Anspruch 11, wobei sich die flossenförmigen Elemente (3) radial vom Boden (12) aus erstrecken.

13. Prothesenschaft (1) nach Anspruch 11 oder 12, der mindestens drei flossenförmige Elemente (3) aufweist.

14. Prothesenschaft (1) nach Anspruch 8 und einem der Ansprüche 11, 12 oder 13, wobei sich das Spannelement (6) zwischen den flossenförmigen Elementen (3) in Höhe der Wandabschnitte (2) erstreckt.

15. Prothesenschaft (1) nach Anspruch 10 und 14, wobei sich der Kanal (5) in einem flossenförmigen Element (3) in Höhe des Wandabschnittes (2) erstreckt.

## Revendications

1. Emboîture de prothèse (1) agencée de manière à assurer la liaison à une prothèse de membre inférieur, dans laquelle l'emboîture de prothèse (1) comprend un réceptacle (11) destiné à recevoir un moignon (100) d'un patient, dans laquelle le réceptacle (11) comprend une partie inférieure (12) destinée à recevoir une extrémité distale du moignon et une paroi latérale interne (31) destinée à envelopper au moins partiellement ledit moignon (100), dans laquelle la paroi latérale interne (31) est définie par une pluralité de parties de paroi (2) et dans laquelle l'emboîture de prothèse (1) comprend un mécanisme de réglage (6, 7) destiné à régler la position relative d'au moins deux des parties de paroi (2), **caractérisée en ce que** l'emboîture de prothèse (1) comprend, en outre, au moins une section flexible (4) reliant une partie de paroi (2) à la partie inférieure (12), dans laquelle la section flexible (4) est plus flexible que la partie inférieure (12) et/ou la partie de paroi (2).

2. Emboîture de prothèse (1) selon la revendication 1, dans laquelle la section flexible (4) est fabriquée à partir du même matériau que l'emboîture (1).

3. Emboîture de prothèse (1) selon la revendication 2, dans laquelle la section flexible (4) est réalisée d'épaisseur plus faible ou formée d'une autre manière de manière à introduire de la flexibilité.

4. Emboîture de prothèse (1) selon la revendication 1, 2 ou 3, dans laquelle le mécanisme de réglage (6, 7) est agencé de manière à régler un diamètre de la paroi latérale interne (31) du réceptacle (11).

5. Emboîture de prothèse (1) selon l'une quelconque des revendications précédentes, dans laquelle le mécanisme de réglage (6, 7) est agencé de manière à régler la position d'une pluralité de parties de paroi (2).

6. Emboîture de prothèse (1) selon l'une quelconque des revendications précédentes, dans laquelle les au moins deux parties de paroi (2) s'étendent approximativement à des emplacements diamétralement opposés de la paroi latérale interne (31).

7. Emboîture de prothèse (1) selon l'une quelconque des revendications précédentes, dans laquelle le mécanisme de réglage (6, 7) peut être actionné manuellement, de préférence, en utilisant un bouton (7).

8. Emboîture de prothèse (1) selon l'une quelconque des revendications précédentes, dans laquelle le mécanisme de réglage (6, 7) comprend un élément de liaison (6) s'étendant entre au moins deux parties de paroi (2), dans laquelle le mécanisme de réglage (6, 7) est agencé de manière à régler la longueur dudit élément de liaison (6).

9. Emboîture de prothèse (1) selon la revendication 7, dans laquelle ledit élément de tension (6) s'étend autour du périmètre du réceptacle (11) et/ou dans laquelle le mécanisme de réglage (6, 7) comprend, en outre, un bouton rotatif (7) destiné à régler la longueur de l'élément de liaison (6).

10. Emboîture de prothèse (1) selon la revendication 8 ou 9, dans laquelle le réceptacle (11) comprend au moins un canal (5) destiné à recevoir l'élément de liaison (6).

11. Emboîture de prothèse (1) selon l'une quelconque des revendications précédentes, dans laquelle l'emboîture (11) comprend une pluralité d'éléments en forme d'ailette (3) reliés à la partie inférieure (12) en utilisant la au moins une section flexible (4), dans laquelle les surfaces internes (31) des éléments en forme d'ailette (3) comprennent les parties de paroi (2).

12. Emboîture de prothèse (1) selon la revendication 11, dans laquelle les éléments en forme d'ailette (3) s'étendent radialement à partir de la partie inférieure (12).

13. Emboîture de prothèse (1) selon la revendication 11 ou 12, comprenant au moins trois éléments en forme d'ailette (3).

14. Emboîture de prothèse (1) selon la revendication 8 et l'une quelconque des revendications 11, 12 ou 13, dans laquelle l'élément de liaison (6) s'étend entre les éléments en forme d'ailette (3) au niveau de l'emplacement des parties de paroi (2).

15. Emboîture de prothèse (1) selon la revendication 10 et 14, dans laquelle le canal (5) dans un élément en forme d'ailette (3) s'étend au niveau de l'emplacement de la partie de paroi (2).
